# EUROPEAN PATENT APPLICATION

(11) **EP 4 487 824 A1**
(43) Date of publication of application: **08.01.2025**
(21) Application number: 23762696.5
(22) Date of filing: 02.02.2023
(51) Int. Cl.: A61F 2/90, A61F 2/966

(54) **STENT FOR FULL LENGTH OF AORTA, STENT KIT, AND STENT DELIVERY SYSTEM**

(30) Priority: 03.03.2022 CN 202220454074 U
(71) Applicant: Shanghai Flowdynamics Medical Technology Co., Ltd, Shanghai 201615 (CN)
(72) Inventor: DING, Jian, Shanghai 201615 (CN)
(74) Representative: Gevers Patents
(86) International application number: PCT/CN2023/074223
(87) International publication number: WO 2023/165295

(57) **Abstract**

The present disclosure relates to a stent for the full length of an aorta, a stent kit, and a stent delivery system. The stent is provided with a stent body and a cavity formed by means of enclosure of the stent body. The stent has a length of 200 mm-600 mm and an outer diameter gradually tapered from 60 mm-30 mm to 30 mm-10 mm; and the stent body is woven of at least two wires with different diameters and is configured to be compressible and extensible at any position in the axial direction of the stent in a released state, wherein a first wire has a diameter of 20 µm-150 µm, the second wire has a diameter of 150 µm-800 µm, and at least one branch stent fixing mechanism is arranged in the cavity.

## Description

### CROSS-REFERENCE TO RELATED APPLICATIONS

This application claims priority to Chinese Patent Application No. 202220454074.2 filed on March 3, 2022, the contents of which are hereby incorporated by reference in its entirety.

### TECHNICAL FIELD

The application relates to a stent, a stent kit and a stent delivery system, and in particular to a stent for a full length of an aorta, a stent kit and a delivery system in a treatment of aortic lesions, such as aortic aneurysms or aortic dissections.

### BACKGROUND

An arterial vessel wall is composed of an intima, a tunica media and an adventitia in tight contact with one other. When an inner wall of the arterial vessel is partially damaged, the tunica media of the arterial vessel wall is gradually peeled off under a strong impact of arterial blood flow, so that blood enters between the tunica media and the adventitia of the vessel wall, to form two lumens, i.e., a true lumen and a false lumen. The most common type is aortic dissection. The aortic dissection weakens the arterial wall, so that there is a risk of rupture of the aortic dissection at any time. Once the dissection ruptures, the patient will die in several minutes.

The aortic dissection is divided into two types, i.e., type A and type B (i.e., Stanford classification) according to a tearing site and an expansion range of the intima. Type A refers to a lesion involving an ascending aorta, which refers to a situation in which a peeling site of the arterial wall starts from the ascending aorta, and the lesion may also occur at an aortic arch or a proximal end of a descending aorta involving the ascending aorta. Type B refers to a situation in which a peeling site of the arterial wall occurs at the descending aorta without extending beyond a proximal end of an opening of a left subclavian artery.

The aortic aneurysm is also a disease in which an aorta is abnormally dilated. Rupture of the aortic aneurysm is also fatal for the patient.

Therefore, early diagnosis and timely treatment of the aortic dissection and the aortic aneurysm are essential.

At present, for the treatment of the aortic dissections or the aortic aneurysms, an interventional therapy with a low cost and a low risk is stent implantation.

A covered stent is commonly used for the treatment of the aortic dissection or the aortic aneurysm. When the lesion site involves the aortic arch and/or the abdominal aorta with branch arterial vessels, in order to prevent the stent from obstructing the branch artery, the conventional practice is to perform the on-site drilling at a site of the covered stent corresponding to an important branch artery. On the one hand, the difficulty of surgery is increased, so that the surgery needs to be performed by an experienced surgeon. On the other hand, once the positioning of the stent is inaccurate during placement, or the stent is displaced during a release thereof, an important branch vessel may be obstructed, which may induce serious consequences. Furthermore, the stent is modified before the surgery, and thus the manufacturer thereof may refuse to provide a warranty service for this reason.

A solution for performing an aortic endovascular intervention by using a dense mesh stent is recently proposed. Unlike the EVAR endovascular intervention, in this solution, a mechanism of mechanically obstructing the false lumen is not used, and the dense mesh stent does not significantly hinder the passage of a blood flow. Instead, in this solution, by creating an obstruction to blood flow at the inner wall of the lesion vessel, the hemodynamics in the false lumen is changed, the pressure in the false lumen is reduced, and the intraluminal thrombus is promoted, thereby achieving the therapeutic purposes. Due to the use of the dense mesh stent, compared with the EVAR endovascular intervention, this solution has advantages of less trauma, rapid recovery and low mortality, and the dense mesh stent does not significantly obstruct the blood flow to the branch arteries, thereby greatly reducing the difficulty of surgery. Therefore, this solution may be performed by a doctor with ordinary experience.

However, the treatment effect of this type of stent is not satisfactory. Since the tear of the inner wall of the blood vessel is not completely obstructed, an ideal intraluminal thrombus cannot be always formed. Furthermore, the existing dense mesh stents are still difficult to be applied to all lesion types of aortic dissection and aortic aneurysm, especially difficult to be applied to type A aortic lesion.

Type A aortic dissection involves the ascending aorta, and an inner diameter of the blood vessel at this site is significantly increased due to the lesion, usually up to 38-55 cm. A dense mesh stent with such a large diameter cannot be radially compressed to a stent with a very small diameter, and thus a thicker delivery system is often needed. **In** this case, the delivery system is difficult to be placed through a femoral artery with a relatively small diameter, even the delivery system is unable to be placed especially in Asian people with relatively thinner blood vessels. **In** order to reduce the diameter of the stent in a compressed state, thinner wires may be used to weave the stent. However, this results in that the radial support force of the stent is insufficient to achieve the therapeutic purposes.

Further, type A aortic dissection often involves a site from the descending aorta to the abdominal aorta. For this situation, a plurality of stents are often needed to be placed in segments, so that the operation time is long, and the number of stents is large, which increases the surgical risks and the surgical costs.

Therefore, for the aortic dissection lesions involving most areas of the aorta, a stent, which is more conducive to the surgical safety and can quickly complete the surgery, is still needed.

### SUMMARY

**In** view of this, a main purpose of the application is to provide a dense mesh stent for a full length of an aorta and a delivery system thereof. This stent has a diameter suitable for delivering the stent through a femoral artery in a delivery state and is configured to treat the aortic dissection or the aortic aneurysm, especially suitable for treating lesions involving the entire aorta. The stent, when released, may be partially compressed and/or stretched axially in cooperation with its delivery system, to adjust its length after a placement to adapt to any patient. **In** addition, by adjusting a degree of partial compression, the stent may effectively obstruct the tear of the inner wall of the blood vessel that forms the aortic dissection, and provide the enhanced radial support force to the vessel wall, thereby achieving the better therapeutic effect.

A further purpose of the application is to provide a stent kit, which includes the above stent and a branch artery stent used in cooperation with the above stent. The stent kit, after released, forms a stable stent assembly, which may provide effective treatment and prevention for lesions that have already formed or may form or worsen in patient's aorta and main branch arterial vessels.

To this end, a first aspect of the application provides a stent for a full length of an aorta. The stent includes a stent body and a cavity formed by the stent body. A length of the stent ranges from 200 mm to 600 mm, and an outer diameter of the stent is gradually reduced from 60-30 mm to 30-10 mm. The stent body is formed by weaving at least two kinds of wires having different diameters, the at least two kinds of wires including first wires and second wires, and the stent body is configured to be compressible and extendable at any position along an axial direction of the stent in a release state. A diameter of each of the first wires ranges from 20 µm to 150 µm, a diameter of each of the second wires ranges from 150 µm to 800 µm, and at least one branch stent fixing mechanism is provided in the cavity.

The stent is a stent used for a full length of an aorta, may have a total length ranging from 400 mm to 600 mm in a natural state, and may be appropriately compressed/stretched during its placement in the blood vessel to obtain a suitable length for different aortic structures of different patients. Further, an inner diameter is gradually reduced from the proximal end of the aorta to the abdominal aorta. Thus, the outer diameter of the stent may also be gradually reduced from 60-30 mm at the proximal end (ascending aorta site) to 30-10 mm at the distal end (abdominal aorta site), to adapt to the physiological structure of the diseased aorta.

The stent, in a release process, is axially compressible and extendable at any position, especially according to a stent delivery system and a stent placement method as described in detail below. Further, unlike the dense mesh stents currently used in the aorta, the stent is formed by weaving at least two kinds of wires having different diameters. On the one hand, when the stent is released in the blood vessel, by partially axial compression and/or extension, the total length of the stent may be adjusted, to adapt to the structures and lengths of the aortic vessels of different patients. On the other hand, the thicker second wires form a basic support skeleton and provide a basic radial support force, and the thinner first wires may effectively fill gaps between the second wires and provide an ancillary support and maintain the morphology of the stent. After the stent is axially compressed, due to the presence of the first wires, the fluid permeability of the compressed portion may be significantly reduced to allow an effective obstruction on a tearing site of an inner wall of the blood vessel to be formed. In addition, after the thicker second wires are axially compressed, the density of the second wires per unit length is increased, so that a significantly enhanced radial support force may be formed, thereby expanding the true lumen of the blood vessel and facilitating the reduction of the false lumen. Thus, the wires of the stent having two different diameter ranges cooperate with each other, thereby achieving the better therapeutic effects. On the other hand, due to the above characteristics, the stent may be axially extended to allow the density of the woven wires to be significantly reduced, and then the stent may be radially compressed, so that the stent is easily compressed into a delivery configuration with a suitable diameter, and may be conveniently assembled into the delivery system as described in detail below. Thus, although the stent for the full length of the aorta includes a proximal end portion, of which an outer diameter may be up to 50-60 mm after the stent is released, a delivery device with a conventional diameter (for example, 5-10 mm) may still be used for delivery. Further, due to the above characteristics of the stent, any aperture of the stent is easily expended, so that at a site where the branch artery is present, if necessary, the branch stent may be placed through an aperture located at any suitable position. Further, a tail portion of the branch stent is firmly fixed in the cavity of the stent through the branch stent fixing mechanism.

According to an embodiment, in a natural release state (i.e., without subjected to axial compression and without extension), a metal coverage of the stent is at least 30%, and a radial support force of the stent is greater than or equal to 100 N.

According to an embodiment, in the natural release state, the metal coverage of the stent ranges from 30% to 90%, and the radial support force of the stent ranges from 100 N to 600 N. As described above, the stent is partially compressible in the release process, to form a segment with low liquid permeability and a high support force to obstruct a tear of the inner wall of the blood vessel for treatment. Thus, according to an embodiment, in a release and axial maximum compression state, a metal coverage of the stent is at least 80%, and a radial support force of the stent is greater than or equal to 400 N. According to a specific embodiment, in the release and axial maximum compression state, the metal coverage of the stent ranges from 80% to 100%, and the radial support force of the stent ranges from 400 N to 1000 N. After the stent is compressed to the maximum along the axial direction, the stent having the radial support force in the above range may effectively support a narrowed true lumen of the blood vessel. In particular, the radial support force in the preferred range is particularly conducive to a lesion site which has been formed for a certain period of time, where the torn vessel intima becomes hard and thus requires a greater force to be expanded.

Therefore, in an actual application, when the stent is released in the blood vessel (especially the aorta), the stent has different compression ratios along the axial direction thereof. The radial support forces of different portions of the stent may vary in a range of 100-1000 N, and the metal coverage of different portions of the stent may vary in a range of 30%-100%.

The stent needs to have a suitable weaving density. If the weaving is too dense, radial compression may be difficult even by reducing the number of the second wires. On the contrary, if the weaving is too sparse, it is difficult to form effective obstruction on the tearing site of the intima even by axial compression.

Therefore, in an actual application, when the stent is released in the blood vessel, the metal coverage of different portions of the stent may be different depending on different compression/stretching ratios, for example, the metal coverage may vary between a metal coverage in the axial maximum compression state and a metal coverage in the natural release state or in an appropriate stretching state.

Due to the above characteristics of the stent, it is possible to be relatively flexibly adapted to different treatment requirements for the full length of the aortic vessel from the ascending aorta to the abdominal aorta, for example meeting requirements of repairing the tear, expending the true lumen of the blood vessel, reducing the false lumen, and maintaining a smooth blood flow in the branch artery, simultaneously.

According to an embodiment, the branch stent fixing mechanism for fixing a branch stent is provided in the cavity of the stent and is located at a segment corresponding to a main branch vessel close to an aortic arch and/or an abdominal aorta. In particular, the branch stent fixing mechanism is provided with at least one circular hole, which matches with an outer diameter of a fixing part of a branch stent in a stent kit as described in detail below. The circular hole is substantially perpendicular to an inner wall of the stent body and is provided in the cavity of the stent, so that the branch stent may be firmly engaged with the branch stent fixing mechanism when the fixing part of the branch stent extends into the circular hole. Thus, in this embodiment, preferably, an inner diameter of the circular hole is slightly less than the outer diameter of the fixing part of the branch stent.

According to an embodiment, according to a corresponding site, the branch stent fixing mechanism is provided with two or three circular holes to fix two or three branch stents. More specifically, the circular holes may be arranged tangent to each other.

According to an embodiment, the branch stent fixing mechanism may be integrally woven with the stent body of the stent, to be fixedly connected to the inner wall of the stent body.

According to an embodiment, different segments of the stent may have different metal coverage and/or radial support forces in the above ranges. A variable metal coverage and/or radial support force may be obtained by changing the weaving method of the metal wires, to meet the requirements of different sites of the aorta.

According to an embodiment, in the natural release state, at least part of the segments of the stent has a substantially complete liquid permeability, that is, the blood flow from the aorta to the branch artery is substantially or completely not obstructed. According to this embodiment, in the natural release state, the segment, such as the segment corresponding to the descending aorta and/or the segment corresponding to the abdominal aorta, may have a metal coverage of 30%-60% and a radial support force of 200-600 N. The stent having the radial support force and the metal coverage in above specified ranges has a relatively low radial support force and a relatively low metal coverage in the natural release state, but has a relatively large compressible ratio. The stent may still create a necessary radial support force and metal coverage, which meet the above provisions, after it is compressed, to effectively obstruct the tear of the inner wall of the blood vessel. **In** the release and axial maximum compression state, the stent has a metal coverage of 80%-90%, and a radial support force of 400-1000 N.

Further, in the natural release state, at least another part of the segments of the stent has a metal coverage of 60%-90%, for example a metal coverage of 70%-90%, and a radial support force of 100-600 N. The stent having the radial support force and the metal coverage in above specified ranges has a relatively high radial support force and a relatively high metal coverage in the natural release state, but has a relatively large extendable ratio. The stent may still be radially compressed to a suitable dimension after it is extended, to be assembled into a suitable delivery system. The stent in this embodiment has a relatively large radial support force and a relatively large metal coverage without compression after it is partially released, thereby facilitating immediate support and expansion of the true lumen of the blood vessel, and obstructing a tear to a certain extent. Further, the radial support force may be further enhanced and the metal coverage may be increased by axially compressing a local segment of the stent, to create the necessary radial support force and metal coverage, which meet the above provisions, thereby obstructing the tear of the inner wall of the blood vessel more effectively. In the release and axial maximum compression state, the stent has a metal coverage of 90%-100%, for example a metal coverage of 90%-95%, and a radial support force of 500-1000 N. Such a segment is specifically suitable for the ascending aorta site and/or the aortic arch site.

According to this embodiment, in the natural release state, the segment of the stent with a higher metal coverage has certain (rather than complete) liquid permeability, that is, this segment forms a certain obstruction of blood flow from the artery to the branch artery. In a specific embodiment, since the stent is axially extendable, suitable blood permeability may be obtained by properly stretching a local segment of the stent corresponding to the branch vessel when the stent is placed. To this end, a corresponding portion of the stent is configured to have an increased diameter relative to an adjacent portion thereof, so that a diameter of this area after it is stretched is suitable for an inner diameter of a lumen, which has a branch vessel, of the blood vessel.

According to yet another embodiment, the stent may further include two bifurcation segments, one of the two bifurcation segments corresponding to a left common iliac artery, and another one of the two bifurcation segments corresponding to a right common iliac artery. The two bifurcation segments include a longer first common iliac artery segment and a shorter second common iliac artery segment. The first common iliac artery segment is ultimately placed in one of the left common iliac artery and the right common iliac artery in which the stent is delivered, and the second common iliac artery segment is ultimately placed in another one of the left common iliac artery and the right common iliac artery.

According to needs, an additional stent may be further placed in the common iliac artery where the second common iliac artery segment is located, to support a longer blood vessel.

According to a preferred embodiment, each of the first wires for the stent may have a diameter of 50-150 µm, and each of the second wires may have a diameter of 150-600 µm. The stent is formed by weaving 48-202 wires, among which 4-32 wires are the second wires, and remaining wires are the first wires.

According to an embodiment, the stent is formed by weaving three kinds of wires having different diameters. The second wires may include first thick wires and second thick wires, the first thick wires and the second thick wires having different diameters. According to a specific embodiment, a diameter of each of the first thick wires may range from 150 µm to 300 µm, and a diameter of each of the second thick wires may range from 300 µm to 600 µm. The stent may be formed by weaving 48-202 wires, among which the second wires include 6-24 first thick wires and 4-24 second thick wires, and remaining wires are the first wires, provided that a sum of the number of the first thick wires and the number of the second thick wires is less than or equal to 30.

According to another embodiment, the stent is formed by weaving three kinds of wires having different diameters. The first wires may include first thin wires and second thin wires, the first thin wires and the second thin wires having different diameters. According to a specific embodiment, a diameter of each of the first thin wires may range from 50 µm to 100 µm, and a diameter of each of the second thin wires may range from 100 µm to 150 µm. The stent is formed by weaving 48-202 wires, among which 4-30 wires are the second wires, and remaining wires are the first wires. The first wires include 32-166 first thin wires and 32-166 second thin wires, provided that a sum of the number of the first thin wires and the number of the second thin wires is less than or equal to 198.

According to yet another embodiment, the stent is formed by weaving four kinds of wires having different diameters, i.e., the first and second thin wires as well as the first and second thick wires as described above.

If the number of the thick wires (i.e., the second wires) is excessive, the stent cannot be effectively compressed to the desired delivery state. However, if the number of the thick wires is too small, the stent cannot provide the expected radial support force even after being compressed, and the expected structure and morphology of the stent cannot be maintained in the release state. Furthermore, the thin wires (i.e., the first wires) fill the gaps between the second wires, and may increase the metal coverage without excessively increasing the support force, thereby effectively obstructing the tear in the blood vessel. However, the amount of first wires should not be too much. If the amount of the first wires is too much, it is still difficult to compress the stent to a delivery state with a suitable diameter.

According to an embodiment in which the stent has a metal coverage of 30%-60% and a radial support force of 200-600 N in the natural release state, the number of wires for weaving the stent may be 48-156, for example 48-128. The number of the second wires may be 4-30, and the remaining wires are the first wires. According to a specific embodiment, the stent is formed by weaving two kinds of first wires and one kind of second wires. 4-30 second wires may be used, and the remaining wires are the first wires. The first wires may include 32-120 first thin wires and 32-120 second thin wires, provided that a sum of the number of the first thin wires and the number of the second thin wires is less than or equal to 152. According to another specific embodiment, the stent is formed by weaving one kind of first wires and two kinds of second wires. The second wires include 6-24 first thick wires and 6-24 second thick wires, and the remaining wires are the first wires, provided that a sum of the number of the first thick wires and the number of the second thick wires is less than or equal to 30.

According to an embodiment in which the stent has a metal coverage of 60%-90% and a radial support force of 100-600 N in the natural release state, the number of wires for weaving the stent may be 96-202, for example 96-156. The number of the second wires may be 4-30, for example 4-24, and the remaining wires are the first wires. According to a specific embodiment, when the thick wires having two different diameters are used, the second wires may include 6-12 first thick wires and 4-12 second thick wires, and the remaining wires are the first wires. If the number of the thick wires (i.e., the second wires) is excessive, the stent cannot be effectively compressed to the desired delivery state. However, if the number of the thick wires is too small, the stent cannot provide the expected radial support force even after being compressed, and the expected structure and morphology of the stent cannot be maintained in the release state. According to yet another specific embodiment, when the thin wires having two different diameters are used, 4-30 wires, for example 4-24 wires are the second wires, and the remaining wires are the first wires. The first wires include 32-120 first thin wires and 32-120 second thin wires, provided that a sum of the number of the first thin wires and the number of the second thin wires is less than or equal to 198.

According to an embodiment, the stent is formed of at least two layers of woven meshes. According to an embodiment, the stent is provided with two to four layers of woven meshes. **In** a case that the stent is provided with multiple layers of woven meshes, the entire stent has the radial support force and the metal coverage as specified above.

A method for weaving the stent is not specifically limited. A conventional overlapping and weaving method may be used (that is, there is no constraint at an intersection between the wires), as long as it may facilitate the axial compression and stretching of the stent.

It should be understood that according to a radial support force range and a metal coverage range required by a specific treatment type, those skilled in the art may select a suitable woven material and determine a reasonable number of layers under specific equipment conditions according to the descriptions herein, further select a suitable diameter, number, or the like of the wires, and determine a suitable weaving solution to obtain a stent having the desired radial support force range and metal coverage range. For example, the suitable weaving solution may be designed by dedicated software.

According to a further embodiment, a tip of the stent (especially a proximal end, at which the stent has a maximum radial support force) may be formed in a return weaving manner. As for the other tip of the stent, if there is a burr which cannot be woven in a return weaving manner again, the burr may be positioned on an inner side of the stent by selecting a suitable arrangement of each layer. Alternatively, if there are two layers of burrs, one of the two layers of burrs is woven in a return weaving manner by a small distance, to wrap the other one of the two layers of burrs in the segment which is woven in a return weaving manner. Alternatively, if multiple stents are used in conjunction with each other, a single layer of burr or two layers of burrs may be overlapped with each other in another dense mesh stent. The stent according to this embodiment is provided with a smooth tip, thereby avoiding mechanical damage to the inner wall of the blood vessel by the exposed wire tip (burr).

According to an embodiment, the stent is provided with a first segment arranged at the proximal end, a second segment arranged adjacent to the first segment, and a third segment arranged adjacent to the second segment. The first segment may have a diameter gradually reduced in a range of 55-38 mm, to be suitable for the ascending aorta. A length of the first segment ranges from 8 cm to 11 cm, for example from 8 cm to 10 cm. The second segment may have a diameter gradually reduced in a range of 38-20 mm, to be suitable for a portion in the artery from the aortic arch to the abdominal aorta. A length of the second segment may range from 28 cm to 40 cm, for example from 28 cm to 31 cm. The third segment is provided with a bifurcation. A portion in front of the bifurcation may have a diameter of 20-32 mm. A longer portion (i.e., the first common iliac artery segment) of the bifurcation has a diameter of 10-16 mm and a length of 5-20 cm, to be suitable for a common iliac artery in which the stent is delivered. A shorter portion (i.e., the second common iliac artery segment) of the bifurcation has a diameter of 10-16 mm and a length of 5-20 cm, to facilitate release into the other common iliac artery.

The stent is self-expandable or capsule-expandable. Materials of the first wires and the second wires for weaving the stent may be different, but preferably the same. Generally, the material of the wires may be metal, such as a shape memory alloy (for example, Nitinol), cobalt-chromium alloy, tungsten, or tantalum.

A second aspect of the application provides a stent kit. The stent kit includes the stent for the full length of the aorta as described above, and at least one branch stent for a branch arterial vessel in an aorta.

According to an embodiment, the branch stent is provided with a supporting part and a fixing part. After the branch stent is placed, the supporting part is located in a corresponding branch arterial vessel, to support the corresponding branch arterial vessel, and the fixing part is located in the cavity of the stent and extends through the branch stent fixing mechanism arranged in the cavity, so that the branch stent is fixed relative to the stent for the full length of the aorta.

According to a specific embodiment, the supporting part and the fixing part of the branch stent may have the same diameter, or may have different diameters. More specifically, an outer diameter of the supporting part may match with an inner diameter of a branch vessel. As described above, an outer diameter of the fixing part is slightly greater than the inner diameter of the circular hole of the branch stent fixing mechanism, to firmly engage the branch stent in the branch stent fixing mechanism.

According to a specific embodiment, a side wall of the fixing part of the branch stent is provided with an opening. The opening is configured to allow unhindered passage of blood in the aorta through the fixing part when the branch stent is placed in the branch arterial vessel.

According to another specific embodiment, the supporting part and the fixing part of the branch stent are arranged at a certain angle. The angle is generally consistent with an angle between the branch vessel and the aortic vessel. In this embodiment, an end of the fixing part close to the supporting part is provided with an opening. A projection of the opening overlaps at least partially with the opening at the end of the fixing part, so that an interior of the fixing part forms a through channel.

According to an embodiment, the stent kit may include one to seven branch stents as described above.

A third aspect of the application provides a stent delivery system. The stent delivery system includes a delivery catheter, and the stent for the full length of the aorta as described above.

The delivery catheter includes an outer catheter, an inner catheter and a push rod, in which the outer catheter, the inner catheter and the push rod are arranged coaxially with one another and arranged sequentially from exterior to interior of the delivery catheter. The outer catheter is provided with a proximal end portion and a distal end portion, and the outer catheter is provided with a first hollow cavity penetrating through the outer catheter. The inner catheter extends in the first hollow cavity at the distal end portion of the outer catheter, and the inner catheter is provided with a second hollow cavity penetrating through the inner catheter. The push rod extends through the first hollow cavity of the outer catheter and the second hollow cavity of the inner catheter, and extends out of a distal end of the inner catheter.

The stent is releasably maintained between the push rod and the outer catheter in a delivery configuration and is arranged in the first hollow cavity at the proximal end portion of the outer catheter.

The delivery catheter is configured so that the outer catheter, the inner catheter and the push rod are axially movable relative to one another, and one end of the stent is removably constrained to a proximal end of the push rod, and another end of the stent is removably constrained to a proximal end of the inner catheter.

According to an embodiment, in the stent delivery system, both ends (i.e., the proximal end and the distal end) of the stent are removably constrained to the proximal end of the push rod and the proximal end of the inner catheter, respectively. In this way, when the stent is released, the outer catheter is maintained stationary and the push rod is pushed in a direction toward the heart, so that the push rod may drive the stent and the inner catheter to move in the direction toward the heart, while the outer catheter correspondingly moves in a direction away from the heart, so that the stent starts to be released from the proximal end of the stent.

Since the proximal end of the stent is constrained to the proximal end of the push rod, the stent is not fully expanded and is in a semi-release state during an initial release. At this time, a diameter of the stent has not reached a diameter in a full-release state of the stent, so that the stent does not abut against the vessel wall, allowing the position of the stent to be easily adjusted. Furthermore, for example, the partially released stent may be withdrawn into the delivery catheter, and may be released again after its position is adjusted. Constraint of the proximal end of the stent to the proximal end of the push rod also has another benefit, that is, a frictional resistance between the stent and the outer catheter is reduced during the initial release, since the end of the stent is constrained. Reduction of the frictional resistance is very advantageous for the release of the stent, especially for the release of the stent in the ascending aorta. Since the delivery catheter turns nearly 180° after passing through the aortic arch, a pushing direction of the push rod at the operator side is almost opposite to a movement direction of the push rod at the ascending aorta side. Therefore, the lower the friction between the stent and the outer catheter, the smaller the force used by the operator to push the push rod, and the easier for the operator to control the release process, thereby facilitating the stable release of the stent.

When at least a portion of the stent is released, one end of the stent is maintained stationary, and the other end of the stent is moved toward said one end (for example, the inner catheter and the outer catheter are maintained stationary, and the push rod is pulled back), so that the released portion of the stent may be compressed, thereby obstructing the tear and supporting the true lumen of the blood vessel as described above. Alternatively, one end of the stent is maintained stationary, and the other end of the stent is moved away from said one end (for example, the push rod is maintained stationary, and the inner catheter and the outer catheter are pulled simultaneously), so that the released portion of the stent may be stretched, thereby extending the stent.

Methods for constraining both ends of the stent are not specifically limited, and any existing constraining methods, which may be removed finally, may be used. For example, both ends of the stent may be temporarily fixed to the push rod and the inner catheter by conventional stoppers, respectively.

The stent delivery system may have a conventional outer diameter, thereby facilitating placing, through the femoral artery, the stent at a site in the aorta that needs to be treated. According to the application, even a stent to be released in the ascending aorta, this stent may also be assembled in the delivery catheter by axially extending and radially compressing this stent to a suitable diameter.

According to an embodiment, an outer diameter of the outer catheter of the stent delivery system ranges from about 5 mm to about 10 mm, for example from about 5 mm to about 7 mm.

According to an embodiment, the push rod is hollow for the passage of guide wires.

A placement method for a stent for a full length of an aorta by using the above delivery system may include the following operations. The stent delivery system is guided to a treatment site. At least a portion of the stent is released. One end of the released portion of the stent is maintained stationary, and the stent delivery system is manipulated, so that the other end of the released portion of the stent is moved along an axial direction of the stent to allow the released portion to be compressed/extended. After the stent is completely released, constraints on both ends of the stent are removed.

According to the stent placement method, the position of the stent is adjusted after the stent is released for a certain length, and the push rod may be pulled back in a direction away from the heart when positions of the inner catheter and the outer catheter are fixed and remained unchanged for example (or, the inner catheter and the outer catheter may be pulled in a direction away from the heart when the position of the push rod is fixed and remained unchanged), so that the semi-released stent is driven to be axially compressed (stretched). Such axial compression/stretch is achieved due to the fact that in the stent delivery system, both ends of the stent are constrained, or a portion of the stent has been released and fixed by abutting against the vessel wall, so that when one end is fixed and the other end is moved, the released portion of the stent may be axially compressed/stretched. For a site where a tear of the blood vessel is located, with the stent delivery system, the released segment of the stent may be compressed to its maximum or appropriate metal coverage and its maximum or appropriate radial support force, so that the released segment tightly abuts against the vessel wall at a corresponding site (especially a tearing site of the intima) under the action of its radial support force, thereby completing the release of this segment.

Similarly, other portions of the stent may be selected to be completely released directly as desired, or may be simultaneously released and compressed/stretched in different segments, to adapt to the lesions and different structures and lengths of the aortic vessel of different patients, thereby obtaining the best therapeutic effect. After the final release is substantially completed, constraints on both ends of the stent are removed, and the delivery catheter together with the push rod is withdrawn from the lumen of the blood vessel.

The stent placement method further includes the following operations. It is detected whether a release position is correct after at least the portion of the stent is released. When it is found that the release position is incorrect, the stent delivery system is adjusted so that the released portion is located at a correct position; or the released portion is withdrawn into the delivery catheter, the stent delivery system is adjusted to a correct position, and the stent is released again.

Since in the stent delivery system, both ends of the stent are temporarily constrained, the stent may be compressed or stretched in different segments during the release of the stent. In particular, for the tearing site of the inner wall of the blood vessel, effective obstructing and a desired radial support force may be created at this local area by compression operations, to achieve the best therapeutic effect. In addition, other portions or specific portions of the stent do not obstruct the blood flow, so that there is no risk caused by interruption or deficiency of blood supply to the branch artery.

Optionally, according to an embodiment, in the branch artery area, a larger aperture may also be formed at this local area by stretching operations, to facilitate the placement of the branch stent in the branch vessel. According to another embodiment, since the stent is formed by the overlapping and weaving method, any one of the apertures is expandable, and the branch stent may also be placed directly through the expanded aperture.

According to an embodiment, the method further includes the following operations. The stent delivery system is introduced from a subject's femoral artery, and the delivery catheter is withdrawn from the subject's femoral artery after the stent is placed.

According to an embodiment, the method further includes the following operation. The stent delivery system is guided to the treatment site by the guide wires.

According to an embodiment, the subject is a mammal, in particular a pig or a human, specifically a human, more specifically a yellow race.

According to an embodiment, the method further includes an operation of placement at least one branch stent. In this embodiment, the treatment site further includes a branch vessel.

The application further provides a method for treating a subject's aortic dissection or aortic aneurysm. The method includes the following operations. The stent or the stent and the branch stent in the stent kit as described above is placed in a treatment site in the aorta of the subject to be treated according to the above stent placement method.

### BRIEF DESCRIPTION OF THE DRAWINGS

FIG. 1 is a schematic view of a stent for a full length of an aortic vessel according to an embodiment of the application and a schematic view of a full length of an aortic vessel to which the stent is applied;
FIG. 2 is a partially enlarged view of the stent shown in FIG. 1;
FIG. 3 is a schematic view illustrating partially axial compression, axial stretching and further radial compression of a stent according to an embodiment of the application;
FIG. 4 is a schematic view of a stent for a full length of an aortic vessel according to another embodiment;

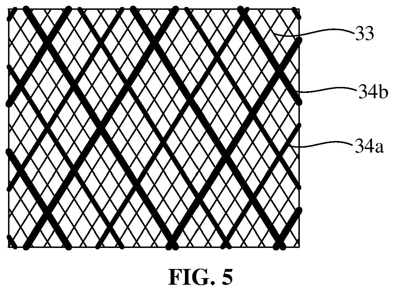

FIG. 5 is schematic partially enlarged view of a stent according to an embodiment of the application;
FIG. 6 is a schematic cross-sectional view of a multi-layer stent wall according to an embodiment of the application;
FIG. 7 is a schematic view of a branch stent in a stent kit according to an embodiment of the application;
FIG. 8 is a schematic view illustrating a placement of a branch stent shown in the part A in FIG. 7;
FIG. 9 shows a stent delivery system according to an embodiment of the application;
FIG. 10 is a schematic view of the stent delivery system shown in FIG. 9 in a semi-release state;
FIG. 11 is a schematic view illustrating placing a stent in type A aortic dissection lesion by using a stent delivery system; and
FIG. 12 is a schematic view illustrating placing a stent at a tear of an aortic intima by using a stent delivery system according to an embodiment of the application.

### DETAILED DESCRIPTION

Technical solutions in embodiments of the application will be clearly and completely described below in combination with the embodiments of the application and the accompanying drawings. It is apparent that the described embodiments are only part of the embodiments of the application, rather than all the embodiments, and the technical solutions recited in the embodiments of the application may be implemented in any combination without conflict. All other embodiments obtained by those of ordinary skill in the art based on the embodiments of the application without any creative efforts belong to the protection scope of the application.

Throughout the description, terms used herein should be understood as meanings as usually used in the art, unless specifically stated otherwise. Therefore, unless defined otherwise, all technical and scientific terms used herein have same meanings as usually understood by those skilled in the art to which the application belongs. When there is a contradiction, meanings of the description are preferred.

Like reference numerals in the accompanying drawings refer to like components. Shapes and dimensions of components in schematic drawings are for illustration only and cannot be considered to reflect actual shapes, dimensions and absolute positions.

It should be noted that in the application, terms "including", "include", or any other variants thereof are intended to cover a non-exclusive inclusion, so that a method or device including a series of elements not only includes elements which are explicitly recited, but also includes other elements which are not explicitly listed, or further includes elements inherent to implementation of the method or device.

It should be noted that terms "first\second" involved in the embodiments of the application are only intended to distinguish similar objects, and do not represent a specific sequence of the objects, and it may be understood that "first\second" may exchange a specific sequence or order in an allowable situation. It should be understood that objects distinguished by "first\second" may be interchanged in an appropriate situation, to enable embodiments and examples of the application described here to be implemented in an order other than those illustrated or described herein.

In order to describe the application more clearly, terms "proximal end" and "distal end" are customary terms used in the field of intervention medical treatment. Herein, "distal end" indicates an end away from the heart during operation, and "proximal end" indicates an end close to the heart during operation.

### STENT

The application provides a stent for a full length of an aorta. The stent is formed by weaving wires having at least two different kinds of diameters, and the stent is configured to be compressible and extendable at any position along an axial direction of the stent.

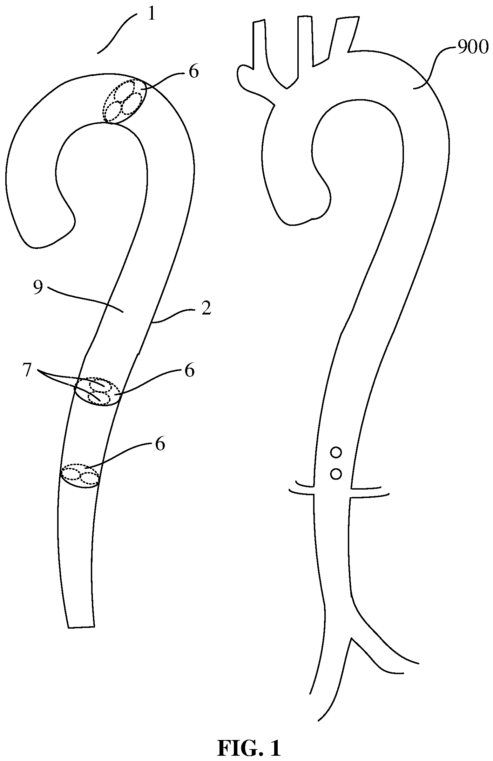

FIG. 1 shows a schematic view of a stent according to an embodiment of the application and a schematic view of an aortic vessel to which the stent is applied. As shown in FIG. 1, the stent 1 is suitable for an aortic vessel 900. The stent 1 includes a stent body 2 and a cavity 9 formed by the stent body 2. The stent 1 shown in FIG. 1 is bent into the same shape as the aorta. In fact, in a case that the stent 1 is not subjected to an external force, the stent 1 is in a straight cylindrical shape with one thicker end and another thinner end.

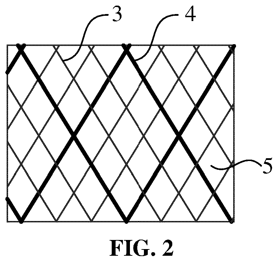

With reference to FIG. 2, FIG. 2 shows an enlarged view of a portion of a stent body of the stent 1 shown in FIG. 1. The stent body 2 of the stent 1 is formed by overlapping and weaving multiple first wires 3 and multiple second wires 4. Therefore, the stent is a bare stent. The portion of the stent body shown in FIG. 2 is a single-layer mesh structure provided with apertures 5.

The stent may also be a structure with multiple layers, such as 2-4 layers. For example, the multiple layers may be formed in a return weaving manner.

The stent is suitable for the entire aortic vessel from the ascending aorta to the abdominal aorta, and thus has a diameter gradually reduced from 50-60 mm (for example, about 55 mm) to 30-10 mm (for example, about 20 mm).

According to the application, the stent 1 may be formed by weaving a total of 48-202 wires. For example, as may be enumerated, the stent may be formed by weaving 48, 64, 96, 128, 156, 196 wires or the like. The number of the wires may be determined according to the diameter of the stent, the number of the layers, the materials of the used wires, or the like.

The material of the stent may be any material suitable for a peripheral vascular stent, as long as the material may provide a sufficient radial support force and have certain fineness. Generally, metal wires such as nickel-titanium alloy wires, cobalt-chromium alloy wires, tungsten wires, tantalum wires, or the like are preferably used, more preferably the nickel-titanium alloy wires.

There are at least four second wires 4 used as thick wires, but the number of the second wires is less than or equal to 32, generally, less than or equal to 30. A diameter of the second wire 4 is comprised between 150 µm and 800 µm, preferably between 150 µm and 600 µm, such as 150 µm, 200 µm, 250 µm, 300 µm, 350 µm, 400 µm, 450 µm, 500 µm, 550 µm, or 600 µm. The second wires 4 provide a basic support force and a complete structure for the stent 1. However, the number of the second wires cannot be too much. For example, even though only wires with the diameter of 300 µm are used, when there are about 32 wires, it is difficult to compress the stent, especially for a stent portion with an ultra-large diameter greater than about 40 mm, to a suitable delivery dimension, so that the stent cannot be used.

In the stent 1, except for the thick wires, the remaining wires are the first wires 3 used as thin wires. A diameter of the first wire 3 may range from 20 µm to 150 µm, preferably from 50 µm to 150 µm, such as 30 µm, 40 µm, 50 µm, 60 µm, 70 µm, 80 µm, 90 µm, 100 µm, 110 µm, 1120 µm, 130 µm, 140 µm and 150 µm. The first wires 3 provide the functions of auxiliary supporting the stent 1 and filling the gaps between the second wires 4. Furthermore, since the number of the first wires 3 is much greater than that of the second wires 4, the first wires 3 also provide a function of maintaining the shape of the stent 1. The inventor has found that although it seems to be feasible theoretically, in fact, with the overlapping and weaving method, a large-diameter stent with a certain shape and a sufficient support force cannot be formed by the second wires (i.e., the thick wires) alone. Furthermore, a large-diameter stent with a fixed wire-to-wire intersection formed by using for example a laser engraving technology, has a much smaller support force, even though the diameter of the wires is the same, which cannot meet the requirements for use in the aortic vessel.

The stent 1 is formed by weaving in an overlapping manner, and an ordered relative movement may happen at an intersection between the wires, so that any aperture 5 on the stent 1 may be easily expanded or compressed.

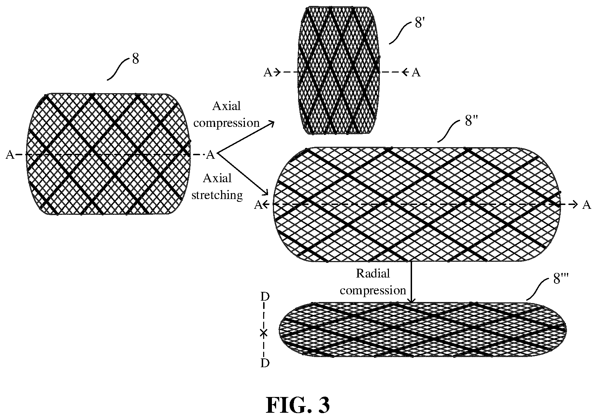

With reference to FIG. 3, FIG. 3 schematically shows that one end of a local segment 8 of the stent 1 is fixed, and the other end of the local segment is moved along a direction of an axis A-A, so that the local segment may be axially compressed into a compressed segment 8', or may be axially stretched into a stretched segment 8".

The "axial direction" of the stent as mentioned herein refers to a direction along A-A as shown in FIG. 3, which is a direction of a central axis of a cylindrical shape of the stent. The "radial direction" of the stent as mentioned herein refers to a direction along D-D as shown in FIG. 3, which is a direction of any diameter of a circle of the cylindrical shape of the stent. In general, "radially compressed" herein refers to compression in a direction from the circumference to the center of the circle.

The above characteristics of the stent in the application may provide many benefits. With reference to FIG. 3, FIG. 3 shows a schematic view illustrating axial compression and axial stretching of the local segment 8 of the stent 1 shown in FIG. 1.

Since the stent may be axially stretched, the density of woven wires, especially the second wires, is significantly reduced per unit length. Therefore, the stretched segment 8" after being properly stretched is easily compressed radially to a delivery configuration 8‴ with a smaller diameter, to be assembled into a delivery system with a conventional outer diameter (for example 5-10 cm, preferably 5-7 cm). Therefore, the stent solves the following problems in the related art: a stent with a large dimension and a high support force cannot be assembled into a delivery system with a suitable diameter.

Furthermore, due to the compressibility and stretchability of the stent along the axial direction, the length of the stent may be adjusted during the placement, so that the stent is suitable for the structure and length of the aorta of any patient.

Most importantly, since the stent may be axially compressed, the compressed stent creates a high metal coverage, and the density of the second wires per unit length is increased to obtain a high radial support force. The high radial support force may achieve an effective expansion of a narrowed part of the blood vessel, and the high metal coverage may achieve very low fluid permeability, thereby effectively obstructing the tear of the vessel intima. In this case, the second wires 4 act as a support skeleton, and the first wires 3 fill the gaps between the second wires 4 to act as a fabric membrane similar to the covered stent. Unlike the covered stent, the stent may provide a relatively high radial support force after being axially compressed, and the stent may even be effectively used for the treatment of type A aortic dissection involving the ascending aorta. Therefore, the stent also solves following problems in the related art: a conventional dense mesh stent or covered stent is difficult to be applied into the ascending aorta. Since any portion of the stent in the application may be compressed, the stent may perform the targeted obstruction during the placement, regardless of the position of the tear causing the aortic dissection.

In a natural release state, a radial support force of the stent 1 may be greater than or equal to 100 N, preferably from 100 N to 600 N. In an axial maximum compression state, a radial support force of the stent 1 may be greater than or equal to 400 N, preferably from 400 N to 1000 N.

The "natural release state" of the stent as mentioned herein refers to a state in which the stent is not axially compressed and released, when it is fixed in a water bath at 37 ± 2°C.

The "axial maximum compression state" of the stent as mentioned herein refers to a state in which the stent is axially compressed until it is unable to be further compressed, when it is in the natural release state.

The "radial support force" of the stent as mentioned herein refers to a force required to compress the stent along a diameter direction to reach 85% of its original diameter, after it is fixed in the natural release state.

Along with the radial support force, the stent also requires a high liquid impermeability, in order to obtain an effect of effectively obstructing the tear of the vessel intima. This property may be represented by the metal coverage. **In** the natural release state, the stent in an embodiment of the application may have a metal coverage of 30%-90%. **In** the axial maximum compression state, the stent in the application may have a metal coverage of 80%-100%, preferably a metal coverage of 80%-95%.

The "metal coverage" of the stent as mentioned herein refers to a metal coverage ratio per unit area calculated by electron microscope scanning.

According to an embodiment, in the natural release state, the stent may have a radial support force greater than or equal to 200 N, and may have a metal coverage of 30%-60%. In this embodiment, the weaving density of the stent is relatively low, so that the radial support force of the stent is also relatively low in the natural release state. In this embodiment, the stent has a high axial compressible ratio, so that the desired radial support force and metal coverage may still be obtained after the stent is compressed. When the stent is placed, a relatively long segment of the stent needs to be released and then compressed, which requires the blood vessel to have a longer straight segment. Thus, the stent is not suitable for a site where a turn is present in the blood vessel, such as the ascending aorta.

According to another embodiment, in the natural release state, the stent may have a radial support force greater than or equal to 100 N, and may have a metal coverage of 60%-90%, preferably a metal coverage of 70%-90%. **In** this embodiment, the weaving density of the stent is relatively high, so that the radial support force of the stent is also relatively high in the natural release state. **In** this embodiment, the stent has a low axial compressible ratio, so that a certain radial support force and metal coverage may be obtained without compression after the stent is released. Thus, the stent is suitable for a site with a shorter straight segment in the blood vessel, such as the ascending aorta.

Thus, according to an embodiment, the stent 1 may have different metal coverage and radial support forces in different segments. This can be achieved through different weaving methods, or by setting different numbers of layers.

With reference to FIG. 1, a plurality of branch stent fixing mechanisms 6 are provided in the cavity 9 of the stent 1. As shown in FIG.1, the branch stent fixing mechanism 6 is provided in the cross-section of the cavity 9, perpendicular to an inner wall of the stent body 2. The position of the branch stent fixing mechanism 6 corresponds to the position of the main branch vessel on an aortic vessel 900, to fix a branch stent placed in the branch arterial vessel. The stent 1 shown in FIG. 1 is provided with three branch stent fixing mechanisms 6, which respectively correspond to three branch arteries of the aortic arch, the left and right renal arteries of the abdominal aorta leading to two kidneys, and the celiac artery and superior mesenteric artery. Certainly, according to specific needs, the number of the branch stent fixing mechanisms may be one to three.

Each branch stent fixing mechanism 6 is provided with two or three circular holes 7. The number of the circular holes 7 is consistent with the number of branch arterial vessels to be further treated near the branch stent fixing mechanism 6. As can be known from the structure of the branch stent described in detail below, an inner diameter of the circular hole 7 in the branch stent fixing mechanism 6 matches an outer diameter of a fixing part of the branch stent (for example, slightly less than the outer diameter of the fixing part of the branch stent), to allow the branch stent to be firmly fixed.

According to the application, the branch stent fixing mechanism 6 is integrally woven with the stent body 2, to be fixedly connected to the inner wall of the stent body 2. The branch stent fixing mechanism 6 may be formed by using the same material and the same weaving method as the stent body 2. Alternatively, unlike the stent body 2, the branch stent fixing mechanism 6 may be woven by using wires with only one diameter. Alternatively, like the stent body 2, the branch stent fixing mechanism 6 may be woven by using wires with different diameters, but may be woven more sparsely to ensure that the branch stent fixing mechanism may be stably fixed on the branch stent and may be compressed to a suitable delivery configuration.

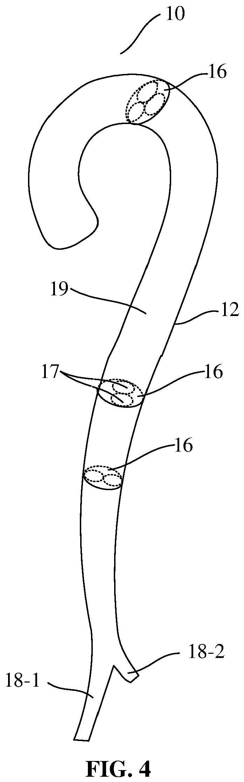

FIG. 4 shows a schematic view of a stent 10 according to another embodiment of the application. The stent 10 has a structure generally the same as the stent 1 shown in FIG. 1: a stent body 12 and a cavity 19 formed by the stent body 12. Three branch stent fixing mechanisms 16 are arranged in the cavity 19 and each branch stent fixing mechanism is provided with circular holes 17 for fixing a branch stent. Unlike the stent 1, the stent 10 is provided, at its distal end portion, with two bifurcation segments respectively corresponding to the left iliac artery and the right iliac artery: a first iliac artery segment 18-1 and a second iliac artery segment 18-2. As shown, it is apparent that the first iliac artery segment 18-1 is longer than the second iliac artery segment 18-2. As can be known from the stent placement method described in detail below, the stent may enter the aorta through the femoral artery for placement by using a delivery system with a conventional dimension. Thus, the first iliac artery segment 18-1 is configured to be suitable for placement in an iliac artery into which the stent enters, and the shorter second iliac artery segment 18-2 is configured to be suitable for placement in the other iliac artery. The specific placement method may be selected according to patient's vascular conditions. According to other embodiments, the first iliac artery segment 18-1 and the second iliac artery segment 18-2 may have the same length.

According to a specific embodiment of the application, a length of the first iliac artery segment 18-1 (the first bifurcation) may range from 50 mm to 200 mm, and a length of the second iliac artery segment 18-2 (the second bifurcation) may range from 50 mm to 200 mm. If the iliac artery in which the second iliac artery segment 18-2 is placed needs to be further treated by placing a stent at its extension portion away from the heart, a suitable stent may be further placed at an end of the second iliac artery segment 18-2 after the stent 10 is placed. The stent to be further placed is not specifically limited in the application, which may be a bare stent or a covered stent.

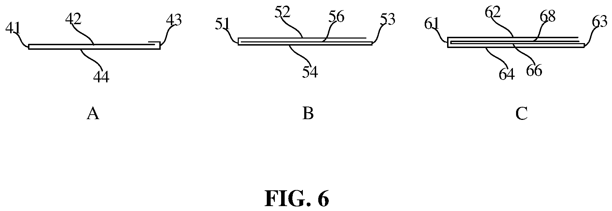

According to an embodiment, the stent may be formed by weaving three or more wires with different diameters. As shown in FIG. 6, a schematic partially enlarged view of a single layer of the stent in this embodiment is shown. The stent is formed by weaving one kind of first wires 33 and two kinds of second wires 34a, 34b. The first wires 33 are thin wires, and may have a diameter of 20-150 µm, preferably a diameter of 50-150 µm. The second wires include first thick wires 34a which may have a diameter of 150-300 µm, and second thick wires 34b which may have a diameter of 300-600 µm. 6-12 first thick wires may be used, more than 4 but no more than 12 second thick wires may be used, and the remaining wires are the first wires. This embodiment may also have other variations, for example, the stent is formed of two kinds of first wires (for example, their diameters are in a range of 20-100 µm and a range of 100-150 µm, respectively) and one kind of second wires, or formed of two kinds of first wires and two kinds of second wires, which is not limited thereto.

A radial support force of the stent formed of three or more kinds of wires with different diameters is more uniform throughout the stent, and the flexibility of the stent may also be enhanced.

As described above, the stent may be provided with multiple layers, preferably two layers, three layers, or four layers. According to a preferred embodiment, the multi-layer stent may be formed by weaving a single-layer woven mesh in a return weaving manner. As shown in parts A to C in FIG. 6, schematic cross-sectional views of walls of stents with two to four layers are shown. The part A in FIG. 6 shows a two-layer structure. An upper layer 42 of the structure in this figure is located at a side of the stent close to the interior of the stent, and a lower layer 44 of the structure is located at a side of the stent close to the exterior (i.e., a side in contact with the vessel wall) of the stent. In this structure, a smooth tip is formed at a proximal end 41 of the structure in a return weaving manner. At a distal end 43 of the structure, the lower layer 44 facing toward the exterior of the stent is woven in a return weaving manner by a certain distance, to wrap an opened edge (burr) at a distal end of the upper layer 42 facing toward the interior of the stent, inside the lower layer 44. In this way, two smooth tips at both ends are formed. Similarly, in the part B in FIG. 6, an upper layer 52 is woven in a return weaving manner at a proximal end 51 to form a lower layer 54, and is further woven in a return weaving manner at a distal end 53 to form an intermediate layer 56. At the distal end 53, the lower layer 54 and the intermediate layer 56 are slightly longer than the upper layer 52, so that an opened edge at the distal end of the upper layer is located inside the stent. In this way, both ends of the stent are also smooth tips. The same principle is also applied to four layers in the part C in FIG. 6. At a distal end 63, two layers 62, 68 close to the interior of the stent are shorter than an edge formed by weaving two layers 64, 66 close to the exterior of the stent in a return weaving manner, while at a proximal end 61, a lowermost layer 64 is formed by weaving an uppermost layer 62 in a return weaving manner, to wrap two intermediate layers 68 and 66 therebetween. One variation may be realized by weaving a small segment at the distal end of one of the uppermost layer 62 or the intermediate layer 68 in a return weaving manner again, to wrap an opened edge of the other one of the uppermost layer 62 or the intermediate layer 68 therebetween. In this way, both ends of the stent are completely smooth tips.

Preferably, the stent is in a multi-layer form, so that both ends (especially the proximal end) of the stent may form smooth tips, to avoid secondary damage to the blood vessel by an opened edge of a braid.

The stent is described in detail as above by way of examples. It should be understood by those skilled in the art that the above examples are intended to explain advantages of the stent, rather than limiting the scope of the application, and features in an example may be applied to the stent in other examples separately or in combination in an appropriate situation. Apparent variations and modifications made to the stent by those skilled in the art according to contents of the application herein, fall within the scope of the application, as long as they meet the concept of the application.

### STENT KIT

The application also provides a stent kit. The stent kit includes the above stent and a branch stent which matches the stent.

The branch stent of the stent kit used in the application may be a stent commonly used in the relate art, as long as a fixing part (or a part retained in the cavity of the aortic stent) of the branch stent has an outer diameter that may match the circular hole of the branch stent fixing mechanism of the stent in the application. The branch stent may also be formed by weaving wires having at least two diameters, or may be formed by weaving wires having only one diameter, which is not specifically limited in the application. Preferably, the material for weaving the branch stent is the same as the material of the stent for the aorta as described above.

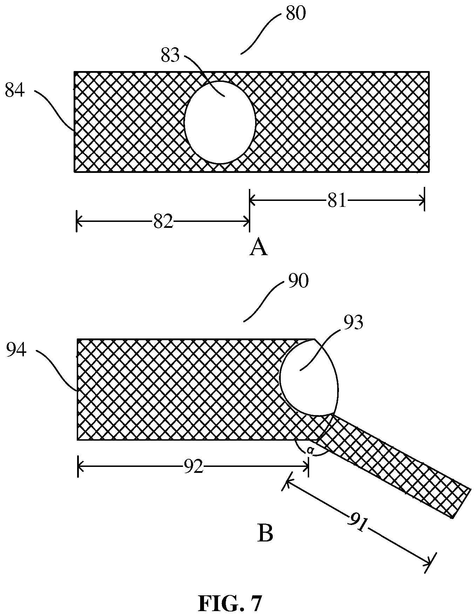

FIG. 7 shows schematic views of two preferred branch stents. As shown in the figure, parts A and B respectively shows two specific embodiments of a branch stent 80 and a branch stent 90 suitable for the stent kit in the application. As shown in the figure, the branch stent 80 and the branch stent 90 are both bare stents, and are formed by weaving metal wires having a single diameter.

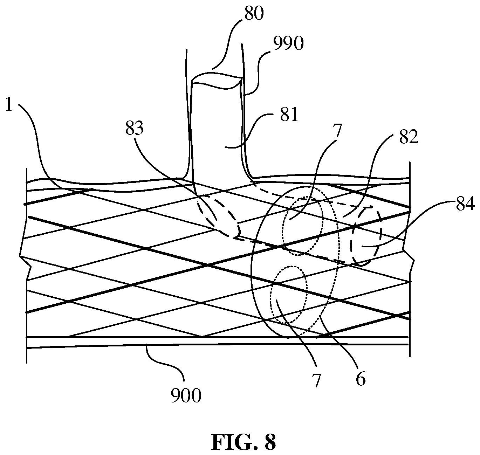

The branch stent 80 shown in the part A in FIG. 7 is provided with a supporting part 81 for supporting a branch vessel, and a fixing part 82 for fixing the branch stent in the branch stent fixing mechanisms 6, 16 arranged in the cavity of the stents 1, 10 for the aorta as described above. The supporting part 81 and the fixing part 82 have the same diameter. A stent wall arranged between these two parts is provided with an opening 83. As further shown in FIG. 8, when the branch stent 80 is placed in combination with the stent 1 (only partially shown) in an aortic vessel 900 with a branch arterial vessel 990, the supporting part 81 passes through an aperture of the stent 1, enters into the branch arterial vessel 990 and supports the branch arterial vessel 990, and the fixing part 82 extends into the cavity of the stent 1 and passes through a circular hole 7 arranged in the branch stent fixing mechanism 6. The opening 83 of the branch stent 80, together with a port 84 formed at an end of the fixing part of the branch stent, allows the fixing part 82 to form a blood flow through path through both ends of the fixing part 82, so that the blood flow in the aorta is smoother.

A branch stent 90 is further shown in the part B in FIG. 7. The branch stent 90 is also provided with a supporting part 91 and a fixing part 92, but a diameter of the supporting part 91 is less than a diameter of the fixing part 92. In this embodiment, the diameter of the supporting part 91 is suitable for an inner diameter of a branch arterial vessel to be supported, and the diameter of the fixing part 92 is suitable for an inner diameter of the circular holes 7, 17 arranged on the branch stent fixing mechanisms 6, 16 in the stents 1, 16 for the aorta. Further, unlike the branch stent 80, there is an angle α between the supporting part 91 and the fixing part 92 of the branch stent 90. The angle α matches with an angle between the branch arterial vessel in which the branch stent is to be placed and the aortic vessel. By dividing the branch stent into two parts with a certain angle therebetween, a restoring force of the bending deformation caused by the placement of the branch stent may be minimized, to better adapt to the structure of the blood vessel. Similar to the branch stent 80, an end of the fixing part 92 of the branch stent 90 connected to the supporting part 91 is also provided with an opening 93. The opening 93, together with a port 94 of the fixing part 92, allows the fixing part 92 to form a through path for the passage of the blood flow.

Certainly, the branch stents in the stent kit are not limited to the specific forms shown in FIG. 7. For example, the branch stent for the stent kit may be in the form shown in part A or part B in FIG. 7, but is not provided with the openings 83 and 93. Alternatively, the branch stent may be a covered stent provided with an opening. Alternatively, the branch stent may be a semi-covered and semi-bare stent, of which the supporting part is covered and the fixing part is not covered.

The number of the branch stents in the stent kit may be multiple, for example, two, three, four, five, six, seven, or even more. Each stent may have different forms to adapt to the structures and treatment requirements of different branch arterial vessels, which is not listed herein.

### STENT DELIVERY SYSTEM AND STENT PLACEMENT METHOD

The application also provides a stent delivery system. In the delivery system, the above-mentioned stent is assembled in the system in a delivery configuration, and both ends of the stent are constrained, and the constraints are removed only after other portions of the stent are released, thereby allowing the stent to be completely released.

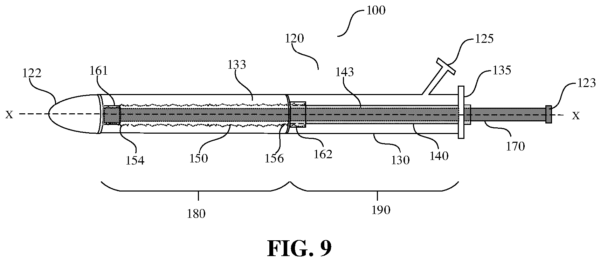

With reference to FIG. 9, a schematic view of a stent delivery system 100 according to the application is shown. The stent delivery system 100 includes a delivery catheter 120 and a stent 150.

The delivery catheter 120 includes an outer catheter 130, an inner catheter 140 and a push rod 170, which are arranged coaxially with each other and sequentially arranged from exterior to interior of the delivery catheter along a longitudinal axis X-X. The delivery catheter 120 is provided with a distal end 123 and a proximal end 122. The delivery catheter 120 is further provided with a hemostatic valve 125. The outer catheter 130 is provided with a proximal end portion 180 and a distal end portion 190, and a first hollow cavity 133 penetrates through the entire outer catheter 130. The inner catheter 140 is arranged coaxially with the outer catheter 130 along the longitudinal axis X-X in the first hollow cavity 133 at the distal end portion 190 of the outer catheter, and the inner catheter is provided with a second hollow cavity 143. The push rod 170 extends through the first hollow cavity 133 of the outer catheter 130 and extends through the second hollow cavity 143 of the inner catheter 140, until extending beyond a port 135 of the distal end of the outer catheter. The push rod 170 may be provided with a third hollow cavity (not shown) for the passage of a guide wire.

At the proximal end portion 180 of the outer catheter 130, the stent 150 is releasably maintained in the first hollow cavity 133 arranged between the push rod 170 and the outer catheter 130 in a delivery configuration. A proximal end 154 of the stent 150 is constrained at a proximal end of the push rod 170 by a first constraint component 161. The first constraint component 161 may be a conventional stopper which may be removed from the stent 150 if necessary, thereby allowing the proximal end 154 of the stent 150 to be released. A distal end 156 of the stent 150 is constrained at a proximal end of the inner catheter 140 by a second constraint component 162. Similarly, the second constraint component 162 may be a conventional stopper which may be removed from the stent 150 if necessary, thereby allowing the distal end 156 of the stent 150 to be released.

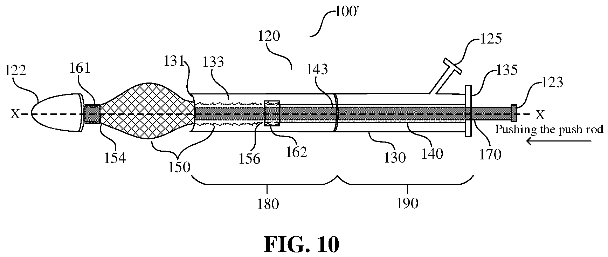

With reference to FIG. 10, a stent delivery system 100' shown in FIG. 9 in a semi-release state is shown. In FIG. 2, a proximal end 131 of the outer catheter 130 in the delivery system 100' may be separated from the proximal end 122 of the delivery catheter 120, and the outer catheter 130 may be moved relative to the inner catheter 140 and the push rod 170 by pushing the push rod 170 toward the proximal end while maintaining the outer catheter stationary (or, by pulling the outer catheter 130 toward the distal end while maintaining the inner catheter and the push rod stationary).

In the system 100' shown in FIG. 10, by pushing the push rod 170 toward the proximal end 122, the proximal end 122 of the delivery catheter 120 drives the push rod 170 fixedly connected thereto, the stent 150 constrained together with an end of the push rod 170 and the inner catheter 140 constrained together with the stent 150 to move toward the proximal end relative to the outer catheter 130. In this way, the stent 150 starts to be released from the proximal end 154 thereof.

Since the proximal end 154 of the stent 150 is constrained during the release, the released segment does not reach its diameter in the natural release state, so that the stent does not contact the vessel wall. On the one hand, it is conducive to adjusting the position of the stent, and on the other hand, the stent 150 may also be withdrawn into the outer catheter 130 again, and then the stent 150 is released again after its position is adjusted. Further, the proximal end 154 of the stent 150 is constrained, which is also conducive to reducing the friction during an initial release and conducive to a stable release. This is particularly important for the surgery remotely performed in a narrow blood vessel.

The stent delivery system is particularly advantageous for the treatment of the type A aortic dissection.

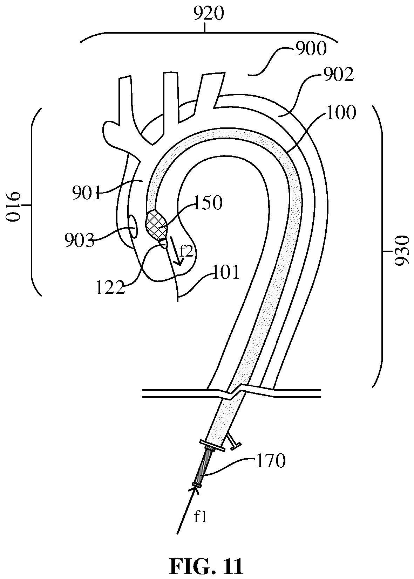

With reference to FIG. 11, a schematic view illustrating placing a stent 150 in type A aortic dissection lesion by using the stent delivery system 100 in an embodiment of the application is shown, in order to explain the therapeutic advantages of the stent delivery system on the type A aortic dissection. FIG. 11 shows that there is a tear 903 in an ascending aorta 910. Thus, a false lumen 902 is formed from the ascending aorta 910, through an aortic arch 920 to a descending aorta 930. The stent delivery system 100 has been guided into a true lumen 901 of the aorta by a guide wire 101. When the stent 150 starts to be released, the operator pushes the push rod 170 in a direction toward the proximal end, where the pushing force is indicated by f1, and a direction of the pushing force is indicated by an arrow directed upward along the descending aorta. The pushing force f1 is transmitted to the proximal end 122 of the delivery catheter along the push rod 170. In this case, since the delivery system 100 turns nearly 180° after passing through the aortic arch 920, a force f2 acting on the proximal end 122 of the delivery catheter has a direction nearly opposite to the direction of the pushing force f1 (as indicated by the arrow). This causes difficulty in controlling a magnitude of the pushing force required during an initial release of the stent. In the stent delivery system 100, with reference to FIG. 10, the proximal end of the stent 150 is temporarily constrained at the proximal end of the push rod 170 by a member, such as a stopper, thereby significantly reducing an outward expansion force of the stent 150 at the proximal end, and reducing the friction between the stent 150 and an inner wall of the outer catheter 130. Therefore, the pushing force required by the delivery system 100 when the stent is initially released is significantly reduced, so that the proximal end of the push rod 170 may be easily pushed out of the outer catheter 130.

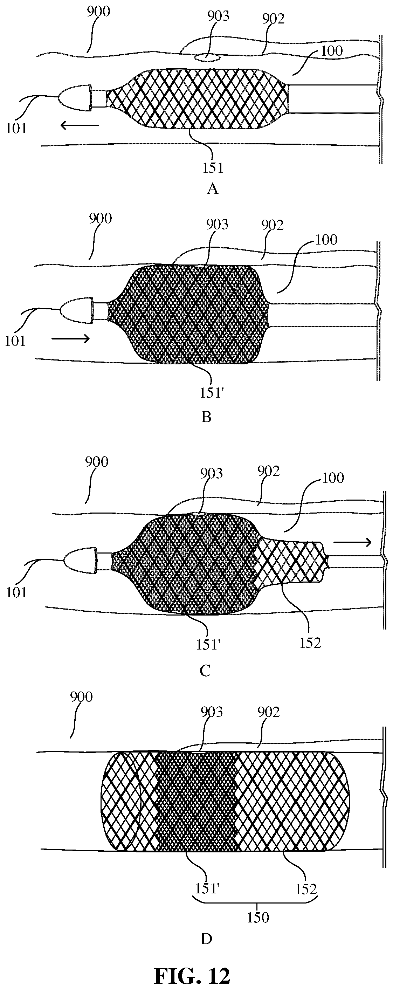

With reference to FIG. 12, a schematic view illustrating placing a stent at a tear of an aortic intima by using a stent delivery system is shown. A segment of aortic vessel 900 including a tear 93 of the vessel intima is shown in the part A in FIG. 12. Tearing of the intima is induced by the tear 903 to form a false lumen 902. The stent delivery system 100 has been guided to the tear 903, and has released a segment 151 of the stent by pushing the push rod in a direction indicated by the arrow in a case that a position of the outer catheter 130 remains unchanged. With reference to the part B in FIG. 12, positions of the inner and outer catheters of the delivery system 100 remain unchanged, and the push rod is pulled back in a direction of the arrow in this figure, so that the released segment 151 of the stent is compressed back and finally abuts against a blood vessel area where the tear 903 of the vessel intima is located, thereby forming a compressed segment 151'. When the stent is designed, the diameter of the stent is usually designed to be slightly greater than the diameter of the blood vessel at a placement site, so that the compressed segment 151' abuts tightly against an inner wall of this segment of the blood vessel, obstructs the tear 903, and expands a true lumen of this segment of the blood vessel. The compressed segment 151' remains in this segment of the blood vessel in the compressed shape, since the compressed segment is subjected to an inward contraction force of the vessel wall.

Next, as shown in the part C in FIG. 12, the remaining segment 152 of the stent is further released by pulling the outer catheter 130 toward the distal end in a direction indicated by the arrow in this figure (the inner catheter 140 and the push rod 170 remain stationary). Finally, after the stent 150 is completely released, constraints of stoppers on both ends of the stent are removed, so that the entire stent 150 is released to the treatment site. Then, the delivery catheter is withdrawn from the blood vessel (see the part D in FIG. 12). The stent 150 placed at the lesion site is provided with two segments, one of the two segments is the compressed segment 151', and the other of the two segments is a natural released segment 152. The compressed segment 151' provides functions of obstructing the tear 903 and providing a relatively strong radial support to the blood vessel. The natural released segment 152 provides a function of supporting other parts of the blood vessel properly, and does not hinder the blood flow, and in particular does not hinder the blood flow toward the branch vessel.

According to the application, after the segment 151 of the stent is released (i.e., a state shown in the part A in FIG. 12), the position of the stent may be confirmed, so that the tear 903 may be accurately obstructed by the compressed segment. If the position is not ideal enough, an adjustment may be performed, or even the released segment 151 may be withdrawn into the outer catheter again, and be re-released after the position of the delivery system is adjusted.

Likewise, the position of the stent may be confirmed after any segment is released, to achieve a best placement effect. Finally, the position of the stent is reconfirmed before the constraints on both ends of the stent are removed, since the stent may be withdrawn and rereleased when its placement position is found to be not ideal at this time. After the constraints on both ends of the stent are removed, the position of the stent cannot be adjusted.

Furthermore, other segments of the stent are compressed in a similar manner to that shown in the part B in FIG. 12. For example, when a front portion of the stent is released and has abutted against the vessel wall, a segment of the stent continues to be released. Since an end of the subsequently released stent is constrained by the outer catheter, the push rod may remain stationary, and the outer catheter and the inner catheter may be moved simultaneously toward the proximal end, so that a new segment of the stent is released. However, a segment of the stent, which has not abutted against the vessel wall yet, is compressed and abuts against the vessel wall, to form a segment with a high metal coverage and a high radial support force.

Detailed solutions of the stent delivery system and the method for placing the stent into the blood vessel by using such a system are explained by the above examples. Variations and modifications may be readily made by those skilled in the art based on the above contents, to be adapted to actual application requirements without departing from the spirit of the application, these variations and modifications also fall within the scope of the application.

### First embodiment

This embodiment provides a stent provided with a structure shown in FIG. 1 and used in an area of the descending aorta. The stent is made of a nickel-titanium alloy material, and is formed by interleaving 54 first wires with a diameter of 100 µm and 12 second wires with a diameter of 400 µm. The stent is woven into two layers in a return weaving manner. The return weaving manner is shown in the part A in FIG. 6. In this figure, the proximal end is a smooth tip which is woven in a return weaving manner. The distal end is two layers of burrs. The burr at the outer side is woven in a return weaving manner by a small segment, to place the burr at the inner side inside the segment which is woven in a return weaving manner, thereby preventing the burrs from being exposed, which damages the vessel wall.

The stent has a conical shape, a diameter of the proximal end of the stent is 45 mm, a diameter of the distal end of the stent is 32 mm, and a length of the stent is 8 cm. A fixed length of this stent after it is released in the blood vessel may be up to 24 cm.

As detected by a scanning electron microscope, a metal coverage of the stent in this embodiment in a natural state is 40%, and a metal coverage of the stent after axial maximum compression is about 90%. As detected by a radial support force tester, a radial support force of a thicker portion of the stent in this embodiment in the natural state is 350 N, and a radial support force of each portion of the stent after the axial maximum compression is greater than 400 N, even greater than 600 N.

In addition, in order to simulate a situation of axial bending stress of the stent when the stent is fixed at the aortic arch, a radial return force of the stent is measured between 0.4 N and 1.0 N.

### Second embodiment

This embodiment provides a stent provided with a structure shown in FIG. 3 and used in an area from the ascending aorta to the aortic arch. The stent is made of a nickel-titanium material, and is formed by interleaving 120 first wires with a diameter of 100 µm and 8 second wires with a diameter of 400 µm. The stent is woven into four layers in a return weaving manner. The return weaving manner is shown in the part C in FIG. 6. **In** this figure, the proximal end is a smooth tip which is woven in a return weaving manner completely. At the distal end, two layers of burrs are located at the inner side of the stent, and two layers of edges, which are woven in a return weaving manner, are located at the outer side of the stent, thereby preventing the burrs from being exposed, which damages the vessel wall.

A portion of the stent corresponding to the ascending aorta has a diameter of 45 mm and a length of 9 cm, and a portion of the stent corresponding to the aortic arch has a diameter of 32 mm and a length of 6 cm.

As detected by a scanning electron microscope, a metal coverage of the stent in this embodiment in a natural state is 80%, and a metal coverage of the stent after axial maximum compression is 98%. As detected by a radial support force tester, a radial support force of a thicker portion of the stent in this embodiment corresponding to the ascending aorta in the natural state is about 400 N, and a radial support force after the axial maximum compression is greater than 400 N, even up to 500 N or more.

**In** addition, in order to simulate a situation of axial bending stress of the stent when the stent is fixed at the aortic arch, a radial return force of the stent is measured between 0.4 N and 1.0 N.

The forgoing is only part of specific embodiments of the application and is not intended to limit the patent scope of the application. Any equivalent structural transformation made by the description and accompanying drawings of the application within the concept of the application, or directly/indirectly applied to other related technical fields, is included within the patent scope of protection of the application.

## Claims

1. A stent for a full length of an aorta, comprising a stent body and a cavity formed by the stent body, wherein a length of the stent ranges from 200 mm to 600 mm, and an outer diameter of the stent is gradually reduced from 60-30 mm to 30-10 mm, wherein the stent body is formed by weaving at least two kinds of wires having different diameters, the at least two kinds of wires comprising first wires and second wires, and the stent body is configured to be compressible and extendable at any position along an axial direction of the stent in a release state, wherein a diameter of each of the first wires ranges from 20 µm to 150 µm, a diameter of each of the second wires ranges from 150 µm to 800 µm, and at least one branch stent fixing mechanism is provided in the cavity.

2. The stent according to claim 1, wherein in a natural release state, a metal coverage of the stent is at least 30%, and a radial support force of the stent is greater than or equal to 100 N, preferably, the metal coverage of the stent ranges from 30% to 90%, and the radial support force of the stent ranges from 100 N to 600 N.

3. The stent according to claim 2, wherein in a release and axial maximum compression state, a metal coverage of the stent is at least 80%, and a radial support force of the stent is greater than or equal to 400 N, preferably, in the release and axial maximum compression state, the metal coverage of the stent ranges from 80% to 100%, and the radial support force of the stent ranges from 400 N to 1000 N.

4. The stent according to claim 1, wherein the branch stent fixing mechanism is provided in the cavity of the stent and is located at a segment corresponding to a main branch vessel close to an aortic arch and/or an abdominal aorta, and the branch stent fixing mechanism is configured to be provided with at least one circular hole for fixing a branch stent, wherein the circular hole is perpendicular to an inner wall of the stent body.

5. The stent according to claim 4, wherein the branch stent fixing mechanism is integrally woven with the stent body.

6. The stent according to claim 1, wherein the stent body is formed by weaving in an overlapping manner to allow each aperture of the stent body to be expendable.

7. The stent according to claim 1, further comprising two bifurcation segments, one of the two bifurcation segments corresponding to a left common iliac artery, and another one of the two bifurcation segments corresponding to a right common iliac artery.

8. The stent according to any one of claims 1 to 7, wherein the diameter of each of the first wires ranges from 50 µm to 150 µm, and the diameter of each of the second wires ranges from 150 µm to 600 µm, and wherein the stent is formed by weaving 48-202 wires, preferably 48-156 wires, more preferably 48-128 wires, among which 4-32 wires are the second wires, and remaining wires are the first wires.

9. The stent according to any one of claims 1 to 7, wherein the at least two kinds of wires having different diameters are three kinds of wires having different diameters, wherein the second wires comprise first thick wires and second thick wires, the first thick wires and the second thick wires having different diameters, a diameter of each of the first thick wires ranging from 150 µm to 300 µm, and a diameter of each of the second thick wires ranging from 300 µm to 600 µm, and wherein the stent is formed by weaving 48-202 wires, among which the second wires comprise 6-24 first thick wires, preferably 6-12 first thick wires, and 4-24 second thick wires, preferably 6-12 second thick wires, and remaining wires are the first wires, provided that a sum of a number of the first thick wires and a number of the second thick wires is less than or equal to 30.

10. The stent according to any one of claims 1 to 7, wherein the at least two kinds of wires having different diameters are three kinds of wires having different diameters, wherein the first wires comprise first thin wires and second thin wires, the first thin wires and the second thin wires having different diameters, a diameter of each of the first thin wires ranging from 50 µm to 100 µm, and a diameter of each of the second thin wires ranging from 100 µm to 150 µm, and wherein the stent is formed by weaving 48-202 wires, among which 4-30 wires are the second wires, and remaining wires are the first wires, the first wires comprise 32-166 first thin wires, preferably 32-120 first thin wires, and 32-166 second thin wires, preferably 32-120 second thin wires, provided that a sum of a number of the first thin wires and a number of the second thin wires is less than or equal to 198, preferably less than or equal to 152.

11. The stent according to claim 1, wherein the stent body is provided with one to four layers of woven meshes, preferably, an end of the stent body is provided with two to four layers of woven meshes.

12. The stent according to claim 1, wherein a material of each of the first wires and the second wires is a biomedical metal or alloy.

13. A stent kit for a full length of an aorta, comprising the stent according to any one of claims 1 to 12 and at least one branch stent.

14. The stent kit according to claim 13, wherein the branch stent is provided with a supporting part and a fixing part, wherein the supporting part is configured to be located in a corresponding branch arterial vessel after the branch stent is placed, to support the corresponding branch arterial vessel, and the fixing part is configured to be located in the cavity of the stent and fixed by the branch stent fixing mechanism after the branch stent is placed.

15. The stent kit according to claim 14, wherein a side wall of the fixing part of the branch stent is provided with an opening, wherein the opening is configured to allow unhindered passage of blood in the aorta through the fixing part when the branch stent is placed in the branch arterial vessel.

16. A stent delivery system, comprising:
a delivery catheter, the delivery catheter comprising an outer catheter, an inner catheter and a push rod, wherein the outer catheter, the inner catheter and the push rod are arranged coaxially with one another and arranged sequentially from exterior to interior of the delivery catheter,
wherein the outer catheter is provided with a proximal end portion and a distal end portion, and the outer catheter is provided with a first hollow cavity penetrating through the outer catheter;
wherein the inner catheter extends in the first hollow cavity at the distal end portion of the outer catheter, and the inner catheter is provided with a second hollow cavity penetrating through the inner catheter;
wherein the push rod extends through the first hollow cavity of the outer catheter and the second hollow cavity of the inner catheter, and extends out of a distal end of the inner catheter; and
wherein the stent delivery system further comprises the stent according to any one of claims 1 to 12, the stent being releasably maintained between the push rod and the outer catheter in a delivery configuration and being arranged in the first hollow cavity at the proximal end portion of the outer catheter.

17. The stent delivery system according to claim 16, wherein an outer diameter of the stent delivery system ranges from 5 mm to 10 mm, preferably from 5 mm to 7 mm.
